# EUROPEAN PATENT APPLICATION

(11) **EP 1 707 637 A1**
(43) Date of publication of application: **04.10.2006**
(21) Application number: 04820283.2
(22) Date of filing: 09.12.2004
(51) Int. Cl.: C12Q 1/26, C12N 9/99, C12N 15/09, G01N 33/15, G01N 33/50

(54) **METHOD OF JUDGING CLINICAL MALIGNANCY OF FALS**

(30) Priority: 11.12.2003 JP 2003413629
(71) Applicant: HISAMITSU PHARMACEUTICAL CO., INC., Tosu-shi, Saga 841-0017 (JP); CHIBA PREFECTURE, Chiba-shi Chiba 260-8667 (JP)
(72) Inventor: NAKAGAWARA, Akira, Chiba Cancer Ctr Research Inst, Chiba-shi, Chiba 2600801 (JP); MIYAZAKI, Kou, c/o Chiba Cancer Ctr Research Inst., Chiba-shi, Chiba 2600801 (JP)
(74) Representative: Grünecker, Kinkeldey, Stockmair & Schwanhäusser Anwaltssozietät
(86) International application number: PCT/JP2004/018410
(87) International publication number: WO 2005/056822

(57) **Abstract**

Clinical malignancy of FALS is determined by isolating mutant SOD1 from a specimen taken from a FALS patient and evaluating the binding ability between the mutant SOD1 and NEDL1 and/or its associated molecule TRAPδ or Dvl1.

SEQUENCE LISTING

<110> Hisamitsu Pharmaceutical Co., Inc.; Chiba-Prefecture

<120> Method for Determining Clinical Severity of FALS

<130> FP04-0444-00WO-HM

<140>
<141>

<150> JP 2003-413629
<151> 2003-12-11

<160> 4

<170> PatentIn Ver. 2.1

<210> 1
<211> 1585
<212> PRT
<213> Homo sapiens

<220>
<223>

<400> 1
Met Ala Ser Pro Ser Arg Asn Ser Gln Ser Arg Arg Arg Cys Lys Glu
1               5                   10                  15

Pro Leu Arg Tyr Ser Tyr Asn Pro Asp Gln Phe His Asn Met Asp Leu
            20                  25                  30

Arg Gly Gly Pro His Asp Gly Val Thr Ile Pro Arg Ser Thr Ser Asp
        35                  40                  45

Thr Asp Leu Val Thr Ser Asp Ser Arg Ser Thr Leu Met Val Ser Ser
    50                  55                  60

Ser Tyr Tyr Ser Ile Gly His Ser Gln Asp Leu Val Ile His Trp Asp
65                  70                  75                  80

Ile Lys Glu Glu Val Asp Ala Gly Asp Trp Ile Gly Met Tyr Leu Ile
                85                  90                  95

Asp Glu Val Leu Ser Glu Asn Phe Leu Asp Tyr Lys Asn Arg Gly Val
            100                 105                 110

Asn Gly Ser His Arg Gly Gln Ile Ile Trp Lys Ile Asp Ala Ser Ser
        115                 120                 125

Tyr Phe Val Glu Pro Glu Thr Lys Ile Cys Phe Lys Tyr Tyr His Gly
    130                 135                 140

Val Ser Gly Ala Leu Arg Ala Thr Thr Pro Ser Val Thr Val Lys Asn
145                 150                 155                 160

Ser Ala Ala Pro Ile Phe Lys Ser Ile Gly Ala Asp Glu Thr Val Gln
                165                 170                 175

Gly Gln Gly Ser Arg Arg Leu Ile Ser Phe Ser Leu Ser Asp Phe Gln
            180                 185                 190

Ala Met Gly Leu Lys Lys Gly Met Phe Phe Asn Pro Asp Pro Tyr Leu
        195                 200                 205

Lys Ile Ser Ile Gln Pro Gly Lys His Ser Ile Phe Pro Ala Leu Pro
    210                 215                 220

His His Gly Gln Glu Arg Arg Ser Lys Ile Ile Gly Asn Thr Val Asn
225                 230                 235                 240

Pro Ile Trp Gln Ala Glu Gln Phe Ser Phe Val Ser Leu Pro Thr Asp
                245                 250                 255

Val Leu Glu Ile Glu Val Lys Asp Lys Phe Ala Lys Ser Arg Pro Ile
            260                 265                 270

Ile Lys Arg Phe Leu Gly Lys Leu Ser Met Pro Val Gln Arg Leu Leu
        275                 280                 285

Glu Arg His Ala Ile Gly Asp Arg Val Val Ser Tyr Thr Leu Gly Arg
    290                 295                 300

Arg Leu Pro Thr Asp His Val Ser Gly Gln Leu Gln Phe Arg Phe Glu
305                 310                 315                 320

Ile Thr Ser Ser Ile His Pro Asp Asp Glu Glu Ile Ser Leu Ser Thr
                325                 330                 335

Glu Pro Glu Ser Ala Gln Ile Gln Asp Ser Pro Met Asn Asn Leu Met
340 345 350.

Glu Ser Gly Ser Gly Glu Pro Arg Ser Glu Ala Pro Glu Ser Ser Glu
355 360 365

Ser Trp Lys Pro Glu Gln Leu Gly Glu Gly Ser Val Pro Asp Arg Pro
370 375 380

Gly Asn Gln Ser Ile Glu Leu Ser Arg Pro Ala Glu Glu Ala Ala Val
385 390 395 400

Ile Thr Glu Ala Gly Asp Gln Gly Met Val Ser Val Gly Pro Glu Gly
405 410 415

Ala Gly Glu Leu Leu Ala Gln Val Gln Lys Asp Ile Gln Pro Ala Pro
420 425 430

Ser Ala Glu Glu Leu Ala Glu Gln Leu Asp Leu Gly Glu Glu Ala Ser
435 440 445

Ala Leu Leu Leu Glu Asp Gly Glu Ala Pro Ala Ser Thr Lys Glu Glu
450 455 460

Pro Leu Glu Glu Glu Ala Thr Thr Gln Ser Arg Ala Gly Arg Glu Glu
465 470 475 480

Glu Glu Lys Glu Gln Glu Glu Glu Gly Asp Val Ser Thr Leu Glu Gln
485 490 495

Gly Glu Gly Arg Leu Gln Leu Arg Ala Ser Val Lys Arg Lys Ser Arg
500 505 510

Pro Cys Ser Leu Pro Val Ser Glu Leu Glu Thr Val Ile Ala Ser Ala
515 520 525

Cys Gly Asp Pro Glu Thr Pro Arg Thr His Tyr Ile Arg Ile His Thr
530 535 540

Leu Leu His Ser Met Pro Ser Ala Gln Gly Gly Ser Ala Ala Glu Glu
545 550 555 560

Glu Asp Gly Ala Glu Glu Glu Ser Thr Leu Lys Asp Ser Ser Glu Lys
565 570 575

Asp Gly Leu Ser Glu Val Asp Thr Val Ala Ala Asp Pro Ser Ala Leu
580 585 590

Glu Glu Asp Arg Glu Glu Pro Glu Gly Ala Thr Pro Gly Thr Ala His
595 600 605

Pro Gly His Ser Gly Gly His Phe Pro Ser Leu Ala Asn Gly Ala Ala
610 615 620

Gln Asp Gly Asp Thr His Pro Ser Thr Gly Ser Glu Ser Asp Ser Ser
625 630 635 640

Pro Arg Gln Gly Gly Asp His Ser Cys Glu Gly Cys Asp Ala Ser Cys
645 650 655

Cys Ser Pro Ser Cys Tyr Ser Ser Ser Cys Tyr Ser Thr Ser Cys Tyr
660 665 670

Ser Ser Ser Cys Tyr Ser Ala Ser Cys Tyr Ser Pro Ser Cys Tyr Asn
675 680 685

Gly Asn Arg Phe Ala Ser His Thr Arg Phe Ser Ser Val Asp Ser Ala
690 695 700

Lys Ile Ser Glu Ser Thr Val Phe Ser Ser Gln Asp Asp Glu Glu Glu
705 710 715 720

Glu Asn Ser Ala Phe Glu Ser Val Pro Asp Ser Met Gln Ser Pro Glu
725 730 735

Leu Asp Pro Glu Ser Thr Asn Gly Ala Gly Pro Trp Gln Asp Glu Leu
740 745 750

Ala Ala Pro Ser Gly His Val Glu Arg Ser Pro Glu Gly Leu Glu Ser
755 760 765

Pro Val Ala Gly Pro Ser Asn Arg Arg Glu Gly Glu Cys Pro Ile Leu
770 775 780

His Asn Ser Gln Pro Val Ser Gln Leu Pro Ser Leu Arg Pro Glu His
785 790 795 800

His His Tyr Pro Thr Ile Asp Glu Pro Leu Pro Pro Asn Trp Glu Ala
805 810 815

Arg Ile Asp Ser His Gly Arg Val Phe Tyr Val Asp His Val Asn Arg
820 825 830

Thr Thr Thr Trp Gln Arg Pro Thr Ala Ala Ala Thr Pro Asp Gly Met
835 840 845

Arg Arg Ser Gly Ser Ile Gln Gln Met Glu Gln Leu Asn Arg Arg Tyr
850 855 860

Gln Asn Ile Gln Arg Thr Ile Ala Thr Glu Arg Ser Glu Glu Asp Ser
865 870 875 880

Gly Ser Gln Ser Cys Glu Gln Ala Pro Ala Gly Gly Gly Gly Gly Gly
885 890 895

Gly Ser Asp Ser Glu Ala Glu Ser Ser Gln Ser Ser Leu Asp Leu Arg
900 905 910

Arg Glu Gly Ser Leu Ser Pro Val Asn Ser Gln Lys Ile Thr Leu Leu
915 920 925

Leu Gln Ser Pro Ala Val Lys Phe Ile Thr Asn Pro Glu Phe Phe Thr
930 935 940

Val Leu His Ala Asn Tyr Ser Ala Tyr Arg Val Phe Thr Ser Ser Thr
945 950 955 960

Cys Leu Lys His Met Ile Leu Lys Val Arg Arg Asp Ala Arg Asn Phe
965 970 975

Glu Arg Tyr Gln His Asn Arg Asp Leu Val Asn Phe Ile Asn Met Phe
980 985 990

Ala Asp Thr Arg Leu Glu Leu Pro Arg Gly Trp Glu Ile Lys Thr Asp
995 1000 1005

Gln Gln Gly Lys Ser Phe Phe Val Asp His Asn Ser Arg Ala Thr Thr
1010 1015 1020

Phe Ile Asp Pro Arg Ile Pro Leu Gln Asn Gly Arg Leu Pro Asn His
1025 1030 1035 1040

Leu Thr His Arg Gln His Leu Gln Arg Leu Arg Ser Tyr Ser Ala Gly
1045 1050 1055

Glu Ala Ser Glu Val Ser Arg Asn Arg Gly Ala Ser Leu Leu Ala Arg
1060 1065 1070

Pro Gly His Ser Leu Val Ala Ala Ile Arg Ser Gln His Gln His Glu
1075 1080 1085

Ser Leu Pro Leu Ala Tyr Asn Asp Lys Ile Val Ala Phe Leu Arg Gln
1090 1095 1100

Pro Asn Ile Phe Glu Met Leu Gln Glu Arg Gln Pro Ser Leu Ala Arg
1105 1110 1115 1120

Asn His Thr Leu Arg Glu Lys Ile His Tyr Ile Arg Thr Glu Gly Asn
1125 1130 1135

His Gly Leu Glu Lys Leu Ser Cys Asp Ala Asp Leu Val Ile Leu Leu
1140 1145 1150

Ser Leu Phe Glu Glu Glu Ile Met Ser Tyr Val Pro Leu Gln Ala Ala
1155 1160 1165

Phe His Pro Gly Tyr Ser Phe Ser Pro Arg Cys Ser Pro Cys Ser Ser
1170 1175 1180

Pro Gln Asn Ser Pro Gly Leu Gln Arg Ala Ser Ala Arg Ala Pro Ser
1185 1190 1195 1200

Pro Tyr Arg Arg Asp Phe Glu Ala Lys Leu Arg Asn Phe Tyr Arg Lys
1205 1210 1215

Leu Glu Ala Lys Gly Phe Gly Gln Gly Pro Gly Lys Ile Lys Leu Ile
1220 1225 1230

Ile Arg Arg Asp His Leu Leu Glu Gly Thr Phe Asn Gln Val Met Ala
1235 1240 1245

Tyr Ser Arg Lys Glu Leu Gln Arg Asn Lys Leu Tyr Val Thr Phe Val
1250 1255 1260

Gly Glu Glu Gly Leu Asp Tyr Ser Gly Pro Ser Arg Glu Phe Phe Phe
1265 1270 1275 1280

Leu Leu Ser Gln Glu Leu Phe Asn Pro Tyr Tyr Gly Leu Phe Glu Tyr
1285 1290 1295

Ser Ala Asn Asp Thr Tyr Thr Val Gln Ile Ser Pro Met Ser Ala Phe
1300 1305 1310

Val Glu Asn His Leu Glu Trp Phe Arg Phe Ser Gly Arg Ile Leu Gly
1315 1320 1325

Leu Ala Leu Ile His Gln Tyr Leu Leu Asp Ala Phe Phe Thr Arg Pro
1330 1335 1340

Phe Tyr Lys Ala Leu Leu Arg Leu Pro Cys Asp Leu Ser Asp Leu Glu
1345 1350 1355 1360

Tyr Leu Asp Glu Glu Phe His Gln Ser Leu Gln Trp Met Lys Asp Asn
1365 1370 1375

Asn Ile Thr Asp Ile Leu Asp Leu Thr Phe Thr Val Asn Glu Glu Val
1380 1385 1390

Phe Gly Gln Val Thr Glu Arg Glu Leu Lys Ser Gly Gly Ala Asn Thr
1395 1400 1405

Gln Val Thr Glu Lys Asn Lys Lys Glu Tyr Ile Glu Arg Met Val Lys
1410 1415 1420

Trp Arg Val Glu Arg Gly Val Val Gln Gln Thr Glu Ala Leu Val Arg
1425 1430 1435 1440

Gly Phe Tyr Glu Val Val Asp Ser Arg Leu Val Ser Val Phe Asp Ala
1445 1450 1455

Arg Glu Leu Glu Leu Val Ile Ala Gly Thr Ala Glu Ile Asp Leu Asn
            1460                1465                1470

Asp Trp Arg Asn Asn Thr Glu Tyr Arg Gly Gly Tyr His Asp Gly His
        1475                1480                1485

Leu Val Ile Arg Trp Phe Trp Ala Ala Val Glu Arg Phe Asn Asn Glu
    1490                1495                1500

Gln Arg Leu Arg Leu Leu Gln Phe Val Thr Gly Thr Ser Ser Val Pro
1505                1510                1515                1520

Tyr Glu Gly Phe Ala Ala Leu Arg Gly Ser Asn Gly Leu Arg Arg Phe
                1525                1530                1535

Cys Ile Glu Lys Trp Gly Lys Ile Thr Ser Leu Pro Arg Ala His Thr
            1540                1545                1550

Cys Phe Asn Arg Leu Asp Leu Pro Pro Tyr Pro Ser Tyr Ser Met Leu
        1555                1560                1565

Tyr Glu Lys Leu Leu Thr Ala Val Glu Glu Thr Ser Thr Phe Gly Leu
    1570                1575                1580

Glu
1585


<210> 2
<211> 6200
<212> DNA
<213> Homo sapiens

<220>
<223>

<400> 2
ggtttttagg cctggccgcc atggcgtctc cttctagaaa ctcccagagc cgacgccggt 60
gcaaggagcc gctccgatac agctacaacc ccgaccagtt ccacaacatg gacctcaggg 120
gcggccccca cgatggcgtc accattcccc gctccaccag cgacactgac ctggtcacct 180
cggacagccg ctccacgctc atggtcagca gctcctacta ttccatcggg cactctcagg 240
acctggtcat ccactgggac ataaaggagg aagtggacgc tggggactgg attggcatgt 300
acctcattga tgaggtcttg tccgaaaact ttctggacta taaaaaccgt ggagtcaatg 360
gttctcatcg gggccagatc atctggaaga tcgatgccag ctcgtacttt gtggaacctg 420
aaactaagat ctgcttcaaa tactaccatg gagtgagtgg ggccctgcga gcaaccaccc 480
ccagtgtcac ggtcaaaaac tcggcagctc ctatttttaa aagcattggt gctgatgaga 540
ccgtccaagg acaaggaagt cggaggctga tcagcttctc tctctcagat ttccaagcca 600
tggggttgaa gaaagggatg tttttcaacc cagaccctta tctgaagatt tccattcagc 660
ctgggaaaca cagcatcttc cccgccctcc ctcaccatgg acaggagagg agatccaaga 720
tcataggcaa caccgtgaac cccatctggc aggccgagca attcagtttt gtgtccttgc 780
ccactgacgt gctggaaatt gaggtgaagg acaagtttgc caagagccgc cccatcatca 840
agcgcttctt gggaaagctg tcgatgcccg ttcaaagact cctggagaga cacgccatag 900
gggatagggt ggtcagctac acacttggcc gcaggcttcc aacagatcat gtgagtggac 960
agctgcaatt ccgatttgag atcacttcct ccatccaccc agatgatgag gagatttccc 1020
tgagtaccga gcctgagtca gcccaaattc aggacagccc catgaacaac ctgatggaaa 1080
gcggcagtgg ggaacctcgg tctgaggcac cagagtcctc tgagagctgg aagccagagc 1140
agctgggtga gggcagtgtc cccgatcgtc cagggaacca aagcatagag ctttccagac 1200
cagctgagga agcagcagtc atcacggagg caggagacca gggcatggtc tctgtgggac 1260
ctgaaggggc tggggagctc ctggcccagg tgcaaaagga catccagcct gcccccagtg 1320
cagaagagct ggccgagcag ctggacctgg gtgaggaggc atcagcactg ctgctggaag 1380
acggtgaagc cccagccagc accaaggagg agcccttgga ggaggaagca acgacccaga 1440
gccgggctgg aagggaagaa gaggagaagg agcaggagga ggagggagat gtgtccaccc 1500
tggagcaggg agagggcagg ctgcagctgc gggcctcggt gaagagaaaa agcaggccct 1560
gctccttgcc tgtgtccgag ctggagacgg tgatcgcgtc agcctgcggg gaccccgaga 1620
ccccgcggac acactacatc cgcatccaca ccctgctgca cagcatgccc tccgcccagg 1680
gcggcagcgc ggcagaggag gaggacggcg cggaggagga gtccaccctc aaggactcct 1740
cggagaagga tgggctcagc gaggtggaca cggtggccgc tgacccgtct gccctggaag 1800
aggacagaga agagcccgag ggggctactc caggcacggc gcaccctggc cactccgggg 1860
gccacttccc cagcctggcc aatggcgcgg cccaggatgg cgacacgcac cccagcaccg 1920
ggagcgagag cgactccagc cccaggcaag gcggggacca cagttgcgag ggctgtgacg 1980
cgtcctgctg cagcccctcg tgctacagct cctcgtgcta cagcacgtcc tgctacagca 2040
gctcgtgcta cagcgcctcg tgctacagcc cctcctgcta caacggcaac aggttcgcca 2100
gccacacgcg cttctcctcc gtggacagcg ccaagatctc cgagagcacg gtcttctcct 2160
cgcaagacga cgaggaggag gagaacagcg cgttcgagtc ggtacccgac tccatgcaga 2220
gccctgagct ggacccggag tccacgaacg gcgctgggcc gtggcaagac gagctggccg 2280

cccctagcgg gcacgtggaa agaagcccgg aaggtctgga atcccccgtg gcaggtccaa 2340
gcaatcggag agaaggtgaa tgtcctatac tccataattc ccagccagta agccagcttc 2400
cttccctgag gcctgaacat catcactacc caacaatcga tgagcctctt ccaccaaact 2460
gggaagctcg aattgacagc cacgggcggg tcttttatgt ggaccacgtg aaccgcacaa 2520
ccacctggca gcgtccgacg gcagcagcca ccccggatgg catgcggaga tcggggtcca 2580
tccagcagat ggagcaactc aacaggcggt atcaaaacat tcagcgaacc attgcaacag 2640
agaggtccga agaagattct ggcagccaaa gctgcgagca agccccagca ggaggaggcg 2700
gaggtggagg gagtgactca gaagccgaat cttcccagtc cagcttagat ctaaggagag 2760
aggggtcact ttctccagtg aactcacaaa aaatcacctt gctgctgcag tccccagcgg 2820
tcaagttcat caccaacccc gagttcttca ctgtgctaca tgccaattat agtgcctacc 2880
gagtcttcac cagtagcacc tgcttaaagc acatgattct gaaagtccga cgggatgctc 2940
gcaattttga acgctaccag cacaaccggg acttggtgaa tttcatcaac atgttcgcag 3000
acactcggct ggaactgccc cggggctggg agatcaaaac ggaccagcag ggaaagtctt 3060
ttttcgtgga ccacaacagt cgagctacca ctttcattga cccccgaatc cctcttcaga 3120
acggtcgtct tcccaatcat ctaactcacc gacagcacct ccagaggctc cgaagttaca 3180
gcgctggaga ggcctcagaa gtttctagaa acagaggagc ctctttactg gccaggccag 3240
gacacagctt agtagctgct attcgaagcc aacatcaaca tgagtcattg ccactggcat 3300
ataatgacaa gattgtggca tttcttcgcc agccaaacat ttttgaaatg ctgcaagagc 3360
gtcagccaag cttagcaaga aaccacacac tcagggagaa aatccattac attcggactg 3420
agggtaatca cgggcttgag aagttgtcct gtgatgcgga tctggtcatt ttgctgagtc 3480
tctttgaaga agagattatg tcctacgtcc ccctgcaggc tgccttccac cctgggtata 3540
gcttctctcc ccgctgttca ccctgttctt cacctcagaa ctccccaggt ttacagagag 3600
ccagtgcaag agccccttcc ccctaccgaa gagactttga ggccaagctc cgcaatttct 3660
acagaaaact ggaagccaaa ggatttggtc agggtccggg gaaaattaag ctcattattc 3720
gccgggatca tttgttggag ggaaccttca atcaggtgat ggcctattcg cggaaagagc 3780
tccagcgaaa caagctctac gtcacctttg ttggagagga gggcctggac tacagtggcc 3840
cctcgcggga gttcttcttc cttctgtctc aggagctctt caacccttac tatggactct 3900
ttgagtactc ggcaaatgat acttacacgg tgcagatcag ccccatgtcc gcatttgtag 3960
aaaaccatct tgagtggttc aggtttagcg gtcgcatcct gggtctggct ctgatccatc 4020
agtaccttct tgacgctttc ttcacgaggc ccttctacaa ggcactcctg agactgccct 4080
gtgatttgag tgacctggaa tatttggatg aggaattcca ccagagtttg cagtggatga 4140
aggacaacaa catcacagac atcttagacc tcactttcac tgttaatgaa gaggtttttg 4200
gacaggtcac ggaaagggag ttgaagtctg gaggagccaa cacacaggtg acggagaaaa 4260
acaagaagga gtacatcgag cgcatggtga agtggcgggt ggagcgcggc gtggtacagc 4320
agaccgaggc gctggtgcgc ggcttctacg aggttgtaga ctcgaggctg gtgtccgtgt 4380
ttgatgccag ggagctggag ctggtgatag ctggcaccgc ggaaatcgac ctaaatgact 4440
ggcggaataa cactgagtac cggggaggtt accacgatgg gcatcttgtg atccgctggt 4500
tctgggctgc ggtggagcgc ttcaataatg agcagaggct gagattactg cagtttgtca 4560
cgggaacatc cagcgtgccc tacgaaggct tcgcagccct ccgtgggagc aatgggcttc 4620
ggcgcttctg catagagaaa tgggggaaaa ttacttctct ccccagggca cacacatgct 4680
tcaaccgact ggatcttcca ccgtatccct cgtactccat gttgtatgaa aagctgttaa 4740
cagcagtaga ggaaaccagc acctttggac ttgagtgagg acatggaacc tcgcctgaca 4800
ttttcctggc cagtgacatc acccttcctg ggatgatccc cttttccctt tcccttaatc 4860
aactctcctt tgattttggt attccatgat ttttattttc aaaccaaatc aggattgaca 4920
aaagctgtgc atgaagaact gccttcttct aagatctaac cttcaggctt ctctcctctg 4980
ttttcaatga actgctagcc tgtatgcaat attaaaaaac agctgtctca aggtctgtgt 5040
atatctccac atacctccat tactaacaat gaaatatgaa tgcaagttaa gctacacttg 5100
accaaatggt aataaatgtt tacttccatt tctatcattg aagggaaaat gtgagcatta 5160
agcactccag gctttcatat gcccatgtct tctgagcaga gccaccattt tttataattt 5220
ctaataacca actccagaac taggagctga tcaactcttt gttttcctct ccatctactt 5280
ttccctgtgc ataatatcca tccaaaggac aacagtggca aagctgaaat ttttatacat 5340
tcaactcatg attcacatgt ggcatcagtc ccatcagccg gaactagcct agacatacgg 5400
tgcaaatatg acacttctaa cgattaacaa cagcaagaaa acacctgctg ctgatgcaat 5460
gcaatgcatc ccaatggttg tggggattgt gggctcaact caagagaagt ttaggagggg 5520
gagcatccct agtgaatact cacaccacaa gaaggacaaa cttgtgcaca tgtccaagaa 5580
agaaagcttc ttgattgagg tagcatgaag gatgaggctt cagcccccat tgtcttatgt 5640
agaatgtggc aatgccaact ggagaaaggg aagaaggaca tattaccttg gtttgaatcc 5700
ctgagttctg tactgttctg ttttgtttag tctagccaca gttcttcaca aaggaaaaaa 5760
aaatgtgtag atgataccat gacttttgtt aaagccatga cttttgtttg cttggcagac 5820
aaaccctttt tttaaaactt tgatattttt ttttcacatt ttttttcctt ttcctttctt 5880
aatcatggag ttcaagttcc tttgcattcg attgtccatc gggaccacac taggaagctg 5940
cagagagtga tggtgcttgt tagggatcaa gggcaacata gtacttctcc ttcacccata 6000
gtaatcctcc tggggcagaa acataacacc ccaaaggcac gttgatttgt atcaaaataa 6060
atatccagtt tcttttagca ttcagtgaaa acatatctca gaaaacttca tgttgtcaga 6120
aaaacagctg caggctccaa agacagccta acctctcaac tacatttgaa ataaacccaa 6180
ccataatggt aaaaaaaaaa 6200

<210> 3
<211> 20
<212> DNA
<213> Artificial Sequence

<220>
<223> Primer

<400> 3
ggtttttagg cctggccgcc                                                    20


<210> 4
<211> 21
<212> DNA
<213> Artificial Sequence

<220>
<223> Primer

<400> 4
caatgaggta catgccaatc c                                                  21

1

## Description

### Technical Field

The present invention relates to a method for determining FALS based on interaction of NEDL1 and its associated factors with mutant SOD1, as well as a therapeutic agent and method therefor.

### Background Art

Amyotrophic lateral sclerosis (ALS) is a neurodegenerative disease with poor prognosis, wherein muscular atrophy occurs by degeneration and deciduation of motor neurons of the spine, motor cortex and brainstem. Familial ALS (hereinafter, "FALS") is currently diagnosed among ALS patients at a frequency of 5-10%, and in some families the Cu/Zn superoxide dismutase (SOD1) gene is the gene responsible, with approximately 20% of FALS cases being attributed to mutations in the SOD1 gene. SOD1 is an active enzyme produced in cells during aerobic metabolism, and it inactivates the superoxide radical. The aggregate theory, which holds that mutated SOD1 (hereinafter referred to as "mutant SOD1") forms aggregates in cells and thereby exhibits cellular toxicity, has recently become the prevailing theory as the cause of FALS (Non-patent document 1).

Upon comparison between neuroblastomas with good prognosis and neuroblastomas with poor prognosis, the present inventors earlier discovered a novel HECT ubiquitin ligase, designated as NEDL1, whose expression is augmented in neuroblastomas with good prognosis (Patent document 1). It was also discovered that NEDL1 ubiquitinates mutant SOD1.

While much still remains to be learned about the intracellular transduction pathway of mutant SOD1, published reports have described the endoplasmic reticulum translocon component TRAPδ (translocon-associated protein complex), as a protein factor which binds solely to mutant SOD1 while not binding to normal SOD 1 (wild-type SOD1) (Non-patent document 2, Non-patent document 3).

This interaction between mutant SOD1, produced by the causative gene for FALS, and its associated molecules are gradually being elucidated, but a full explanation of the mechanism of FALS pathology onset is still far in the future, even if the aforementioned aggregate theory is assumed to be correct.
[Patent document 1] International Patent Publication No. WO 03/018842
[Non-patent document 1] Nakano, R., Saibo Kogaku [Cell Technology], Vol.20, No.11, 1508-1512, 2001.
[Non-patent document 2] Ryen D. Fons, et al, The Journal of Cell Biology, Vol. 160, No. 4, 2003 (529-539)
[Non-patent document 3] Kunst C.B., et al, Nat. Genet. 15, 91-94 (1997)

### Disclosure of the Invention

### Problems to be Solved by the Invention

It has been a goal to elucidate the role of NEDL1 among interaction between mutant SOD1 and its associated peptides, and to verify the aggregate theory as the onset mechanism of FALS. The pursuit of this goal involving evaluation of binding and interaction between different molecules may provide knowledge leading to improved treatment and diagnosis of FALS.

It is one object of the present invention to shed light on binding and interaction between mutant SOD1 and its associated molecules (whether or not mediated by NEDL1) at the genetic or protein level. It is another object of the present invention to apply the acquired knowledge in the clinic, or in other words, to provide a novel therapeutic agent (and method) and diagnostic agent (and method) for FALS.

### Means for Solving the Problems

The present inventors have found that NEDL1 binds to TRAPδ and that these form a complex with mutant SOD1, with a complex binding strength that is roughly proportional to the clinical malignancy of FALS. Similarly, it was found that NEDL1 also binds with Dishevell1 (hereinafter, "Dvl1") and that these form a complex with mutant SOD1, with a complex binding strength that is roughly proportional to the clinical malignancy of FALS. Moreover, it was discovered that interaction between NEDL1 and mutant SOD1 is also roughly proportional to the clinical malignancy of FALS.

Specifically, there is provided a method for determining clinical malignancy of FALS, characterized by isolating mutant SOD1 1 from a specimen taken from a FALS patient and evaluating the binding ability between the mutant SOD 1 and TRAPδ.

There is further provided a method for determining clinical malignancy of FALS, characterized by isolating mutant SOD1 from a specimen taken from a FALS patient and evaluating the binding ability between the mutant SOD 1, and NEDL 1 and Dvl1.

There is still further provided a method for determining clinical malignancy of FALS, characterized by isolating mutant SOD1 from a specimen taken from a FALS patient and evaluating the binding ability between the mutant SOD 1 and NEDL1.

That is, the present invention provides the use of NEDL1 or its substrate for determination of clinical malignancy of FALS. The determination is characterized specifically by the use of isolated mutant SOD1. The aforementioned substrate is preferably TRAPδ or Dvl1.

In addition, there is provided an inhibitor of interaction between mutant SOD 1 and NEDL1 and/or its substrate.

The aforementioned substrate is preferably TRAPδ or Dvl1.

There is even further provided a method of screening for agents that are useful for treatment of FALS, characterized by determining whether or not a candidate drug is an inhibitor against interaction between mutant SOD and NEDL1 and/or its substrate in neurons.

### Effect of the Invention

According to the present invention, it has been demonstrated that complexes are formed between mutant SOD1, which is a causative gene of FALS, and its associated molecules (TRAPδ, Dvl1, etc.) through NEDL 1, and the aggregate theory for the onset mechanism of FALS has been confirmed. Furthermore, since the binding ability between mutant SOD1 and the aforementioned molecules in such complexes (whether or not mediated by NEDL1) are associated with clinical malignancy of FALS, it is possible to determine the clinical malignancy of FALS based on evaluation of the binding ability.

Inhibiting formation of the aforementioned aggregates may contribute to treatment of FALS. According to the invention, therefore, there are provided inhibitors of interaction between mutant SOD1 and NEDL1 and/or its substrate, which should be useful for treatment of FALS.

### Brief Description of the Drawings

Fig. 1 shows a conserved amino acid sequence alignment for human NEDL1 (hNEDL1) and mouse NEDL1 (mNEDL1). The numbers at right represent the amino acid numbers counting from the initiator methionine. The C2 domain, WW domain and HECT domain are all indicated.
Fig. 2 is an electrophoretogram (immunoblot diagram) showing an immunoblot of binding between NEDL1 and TRAP-δ.
Fig. 3 is an electrophoretogram showing an immunoblot of binding between NEDL1 and endogenous TRAP-δ.
Fig. 4 is an immunoblot diagram showing binding between NEDL1 and mutant SOD1.
Fig. 5 is an immunoblot diagram showing ubiquitination of wild-type SOD1 and mutant SOD1 by NEDL 1.
Fig. 6 is an immunoblot diagram showing time-dependent change in degradation of mutant SOD1 and wild-type SOD1 in the presence of NEDL1.
Fig. 7 is an immunoblot diagram showing binding between mutant SOD1 and exogenous TRAPδ.
Fig. 8 is an immunoblot diagram showing binding between NEDL 1 and Dvl1.
Fig. 9 is an immunoblot diagram showing ubiquitination of Dvll by NEDL1.
Fig. 10 is an immunoblot diagram showing time-dependent change in Dvl1 degradation in the presence of NEDL1.
Fig. 11 is an immunoblot diagram showing binding between mutant SOD 1 and Dvl1 in the presence of NEDL1.
Fig. 12 is a schematic diagram showing the interaction between mutant SOD1 and its associated molecules in onset of FALS pathology.

### Best Modes for Carrying Out the Invention

The present invention will now be explained in greater detail with respect to preferred modes.

The NEDL1 gene with which the present invention is concerned is a gene with a total length of 6200 bases (4775-base coding region), and its nucleotide sequence is listed as SEQ ID NO: 2 of the Sequence Listing. The NEDL1 protein encoded by the gene comprises 1585 amino acids, and its full length sequence is listed as SEQ ID NO: 1 of the Sequence Listing. The aforementioned nucleotide sequence and amino acid sequence have been registered with GenBank (http://www.ncbi.nlm.nih.gov.) as Accession No. AB048365.

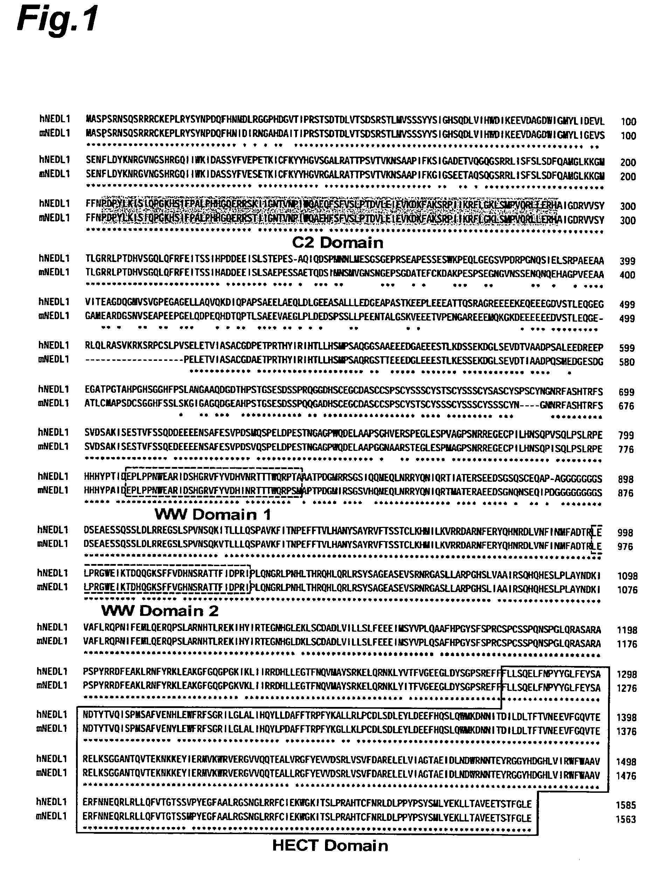

Fig. 1 shows an alignment (homology analysis) for human NEDL1 (hNEDL1) and mouse NEDL1 (mNEDL1). The NEDL-1 protein has the following domains, with HECT ubiquitin ligase features. Specifically, these are (1) an N-terminal C2 domain (which calcium-dependently binds to membrane lipids), (2) a middle WW domain (which contributes to binding with proline-rich regions) and (3) a C-terminal HECT domain (the binding site of ubiquitin-conjugating enzyme E2).

For identification of NEDL1 substrates, the present inventors used the aforementioned WW domain (positions 757-1114) for yeast two hybrid screening. As a result, TRAPδ was discovered as one substrate thereof.

TRAPδ is one constituent protein subunit of translocon-associated protein complex, which is a factor associated with translocation of proteins crossing the endoplasmic reticular membrane. It has already been reported that TRAPδ does not bind wild-type SOD but does bind mutant SOD1 (Kunst C.B. et al., cited above). The present inventors therefore examined interaction between NEDL1, TRAPδ and SOD1. Specifically, we co-transfected COS7 cells with their expression constructs and performed analysis by immunoblotting and immunoprecipitation assay.

### Binding between NEDL1 and TRAPδ

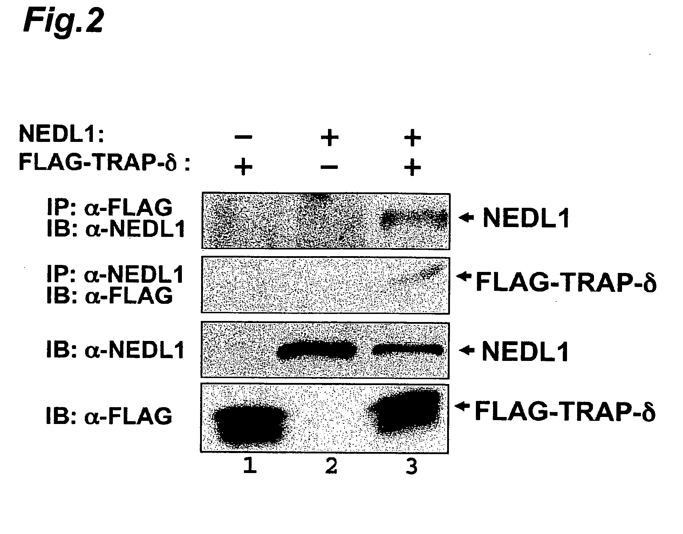

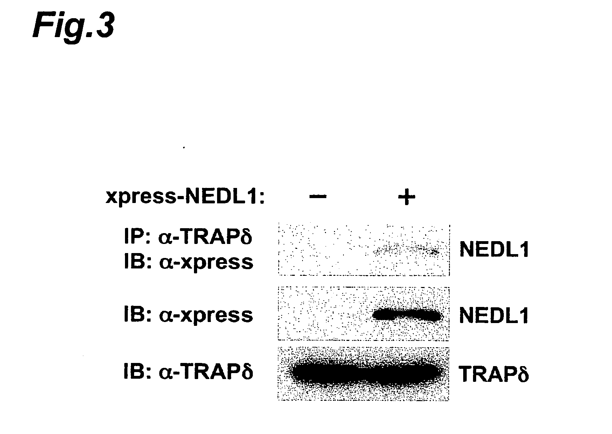

The analysis resulted in confirmation that NEDL1 binds to both endogenous and exogenous TRAPδ, as shown in Figs. 2 and 3. This binding was also confirmed by yeast two-hybrid screening to be through the aforementioned WW domain. However, TRAPδ is not ubiquitinated by NEDL1.

### Binding between NEDL1 and SOD1

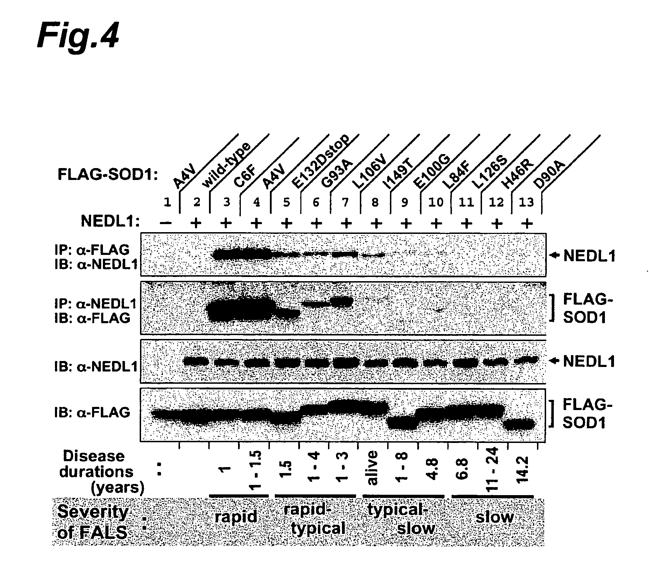

The aforementioned analysis also resulted in confirmation that NEDL 1 binds to mutant SOD1 but not to wild-type SOD1, as shown in Fig. 4. Moreover, the binding ability between NEDL1 and various mutant SOD1 molecules is seen to be roughly proportional to clinical malignancy of the FALS patients from which the mutant SOD1 molecules are isolated.

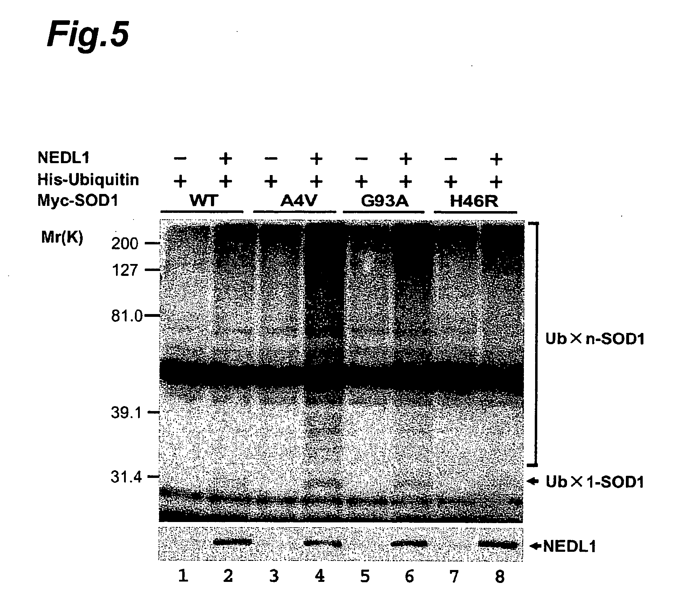

In addition, it was confirmed that NEDL1 ubiquitinates mutant SOD1 (Fig. 5), and that the degree of ubiquitination is roughly proportional to clinical malignancy of the FALS patient from which the mutant SOD1 molecule is isolated.

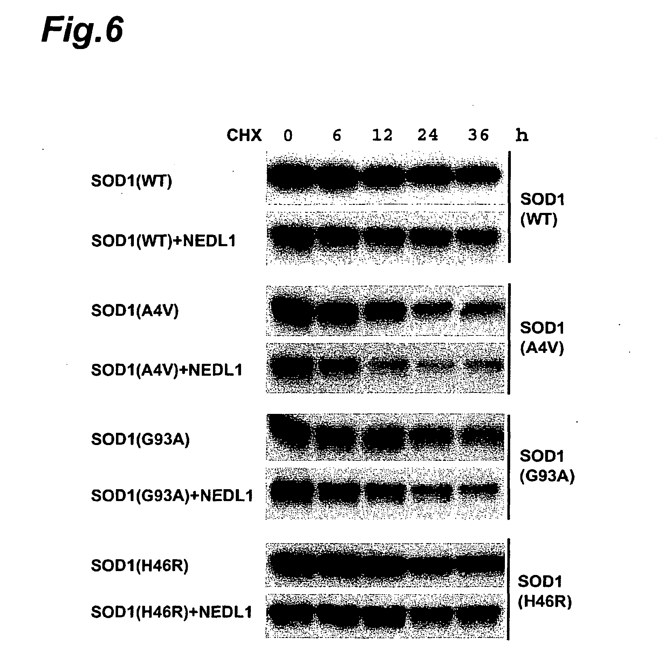

Fig. 6 shows time-dependent change in degradation of mutant SOD 1 in the presence of NEDL1, and here as well it is seen that mutant SOD1 is degraded in proportion to clinical malignancy.

These results indicate that NEDL1 mediates degradation of mutant SOD1 through ubiquitination, and that degradation proceeds in proportion to clinical malignancy.

### Binding between TRAPδ and SOD1

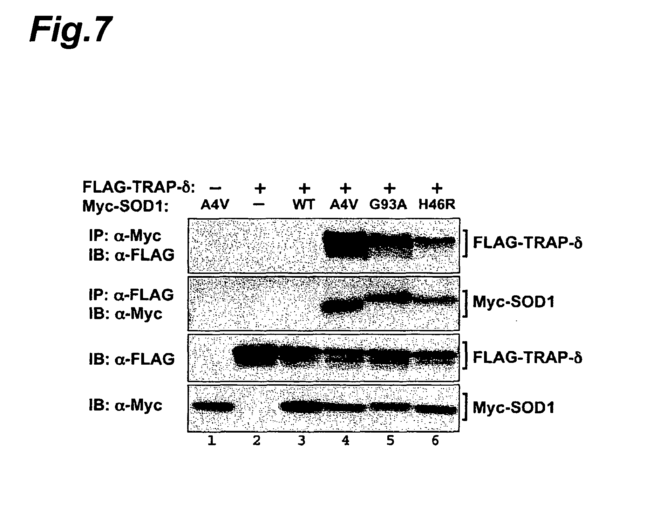

The aforementioned analysis also resulted in confirmation that exogenous TRAPδ binds to mutant SOD1 but not to wild-type SOD1, as shown in Fig. 7. As in the case of NEDL1, the binding ability between TRAPδ and various mutant SOD1 molecules is seen to be roughly proportional to clinical malignancy of the FALS patients from which the mutant SOD 1 molecules are isolated.

To summarize these and other research results, the role of NEDL1 in onset of FALS pathology is presumably the following: (1) NEDL1, either alone or with TRAPδ, ubiquitinates mutant SOD1; (2) NEDL1 and TRAPδ form aggregates with mutant SOD1, which induce fragmentation of the Golgi apparatus and lead to apoptosis of neurons; (3) aggregate formation is responsible for loss of NEDL1 and/or TRAPδ function, producing a condition associated with motor neuron death; and (4) NEDL1/TRAPδ/mutant SOD1 aggregates are taken up and inactivate factors such as molecular chaperones whose normal functioning is important to motor neuron survival.

Next, in order to identify the NEDL1 substrate during ubiquitin-dependent proteolysis, the present inventors performed yeast two hybrid screening using a separate domain of NEDL1 containing the WW domain (positions 382-1448). As a result, Dvl1 was identified as the substrate.

Human DVl1 is a protein comprising 670 amino acids, with the following domains. Specifically, these are (1) the DIX domain required for canonical Wnt/TCF signaling, (2) the PDZ domain (target of Stbm, CKI binding) and (3) the DEP domain (involved in membrane localization during PCP signaling) (D.J. Sussman et al., Dev. Biol. 166, 73-86 (1994); A. Wodarz et al., Cell Dev. Biol. 14, 59-88 (1998); M. Boutros et al" Cell, 94, 109-118 (1998)), and it is believed that the DEP domain binds with the NEDL 1 WW domain.

The interaction between NEDL1, DVl1 and SOD1 was examined, in the same manner as for TRAPδ. Specifically, we co-transfected COS7 cells with their expression constructs and performed analysis by immunoblotting and immunoprecipitation assay.

### Binding between NEDL1 and Dvl1

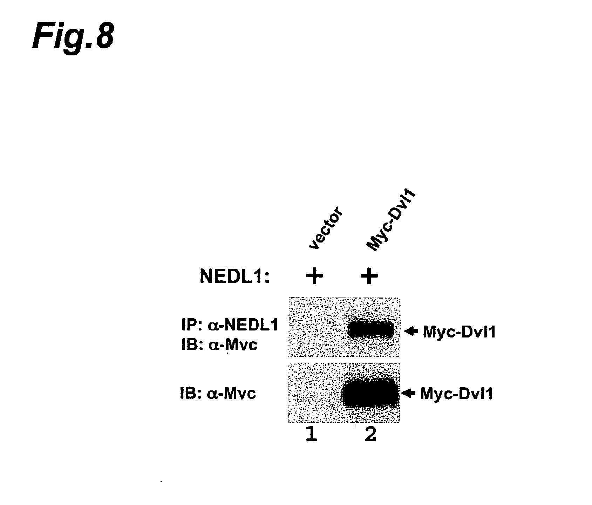

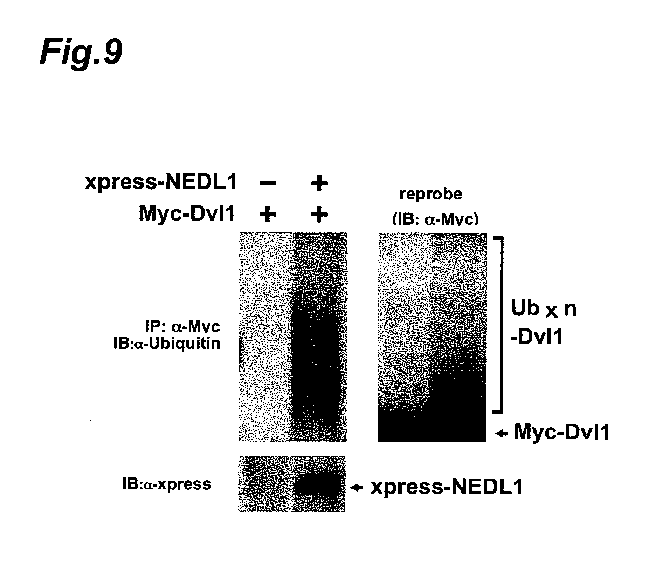

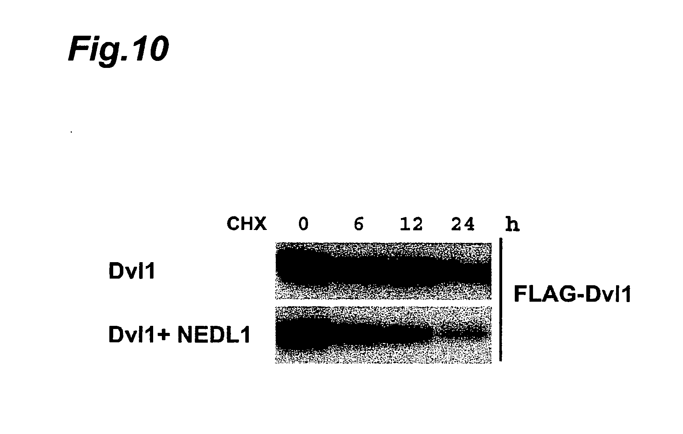

The aforementioned analysis resulted in confirmation that NEDL1 binds Dvl1, as shown in Fig. 8. It was also confirmed that NEDL 1 ubiquitinates Dvl1 (Fig. 9), and Fig. 10 shows time-dependent change in degradation of Dvl1 in the presence of NEDL 1.

### Binding between Dvl1 and SOD1

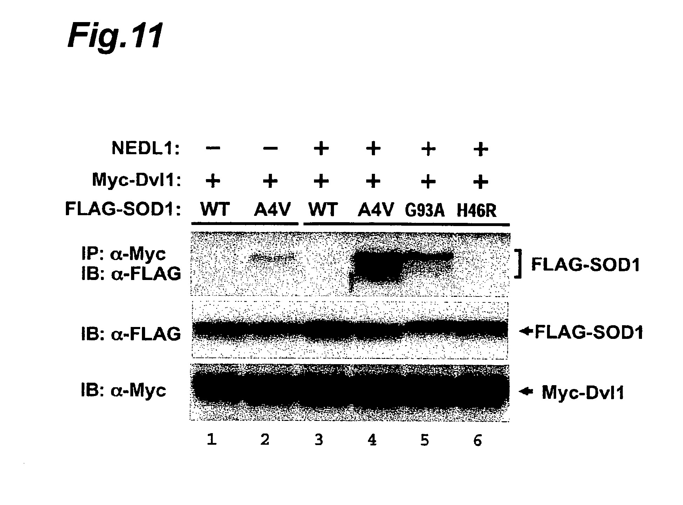

The aforementioned analysis resulted in confirmation that Dvl1 binds SOD 1 in the presence of NEDL1, as shown in Fig. 11. Moreover, the binding ability between Dvl1 and various mutant SOD1 molecules is seen to be roughly proportional to clinical malignancy of the FALS patients from which the mutant SOD1 molecules were isolated.

Motor neuron cytoskeleton abnormalities have been reported in ALS patients, suggesting that the influence of the aforementioned mutant SOD1 molecules on NEDL1-mediated Dvl1 degradation may contribute to motor neuron death (Luo, Z.G et al., Neuron 35, 489-505 (2002)).

### Conclusion

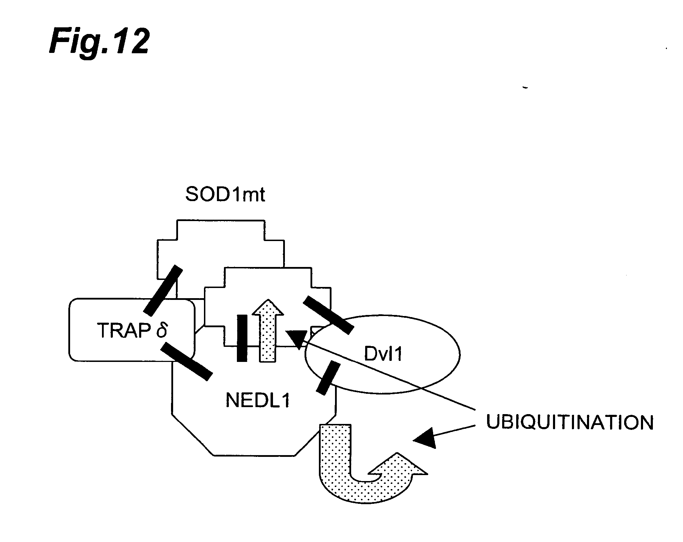

The neuronal E3 ubiquitin ligase NEDL1 binds (interacts with) TRAPδ but also binds Dvl1, ubiquitinating and degrading it. Since NEDL1 thus forms complexes with mutant SOD1, DVl1 and TRAPδ, it forms large aggregates particularly with mutant SOD1 molecules that have escaped ubiquitin-mediated degradation. The function of NEDL 1 that affects the activity of the target protein (substrate) DVl1 or TRAPδ is regulated by mutant SOD1. All of these individual interactions are connected with onset of FALS pathology. Thus, elucidation of the molecular mechanisms by which these complexes or large aggregates are formed will shed light on motor neuron death in ALS and should thus lead to promising new therapeutic agents and methods for ALS. Fig. 12 is a schematic diagram showing the interaction between mutant SOD1 and its associated molecules in onset of FALS pathology, based on knowledge obtained according to the present invention.

### FALS therapeutic agent and treatment method

In light of the aforementioned considerations, the present invention provides inhibitors against interaction between mutant SOD1 and NEDL1 and/or its substrate. Candidate drugs that are potentially useful as inhibitors include nucleic acids, proteins, low molecular compounds (chemically synthesized or naturally derived), and polymer compounds other than proteins.

As a screening method for such inhibitors there may be mentioned the Two-Hybrid System (for example, Gyuris, J. Cell, 1993, 75, 791-803; Golemis, E. A., Current Protocols in Molecular Biology (John Wiley & Sons, Inc.) 1996, Ch. 20.0-20.1). The inhibitor screening method may also be carried out by an immunological method. Specifically, after expressing a mutant SOD 1 (especially one associated with high clinical malignancy) and NEDL1 and/or its substrate in cells (neurons) and culturing the cells for a fixed period with the candidate drug, the cells are crushed to prepare a cell lysate. By immunoprecipitating one molecule with an antibody and detecting or quantifying the other (different) molecule in the precipitate using an immunological method, it is possible to detect the effects of candidate drugs on the interaction of each molecule. Screening of inhibitors is also possible by simultaneously adding an appropriate agonist with the candidate drug to the culturing system and performing the aforementioned assays, and then comparing this with immunoprecipitate from cells containing no candidate drug.

The protein or other inhibitor of mutant SOD1 and NEDL1 and/or its substrate, once identified by the aforementioned screening method, is then orally or parenterally administered to a patient suffering from or suspected of suffering from FALS. The protein is formulated as a pharmaceutical preparation for this purpose. An appropriate dosage form is prepared by combining an effective dose of the binding inhibitor with a pharmaceutically acceptable carrier or diluent. Dosage forms suitable for administration include tablets, pills, powders, solutions, suspensions, emulsions, capsules, suppositories, injections and the like.

### Examples

### (Method)

### (Cloning of human NEDL cDNA)

Cloning of human NEDL 1 cDNA is described in detail in International Patent Publication No. WO 03/018842 (PCT/JP02/08524), but for the present invention it was carried out in the following manner. Specifically, a forward primer (5'-GGTTTTTAGGCCTGGCCGCC-3', SEQ ID NO: 3) and a reverse primer (5'-CAATGAGGTACATGCCAATCC-3', SEQ ID NO: 4) were used for amplification of the 5' portion of NEDL1 cDNA (from a human neuroblastoma cDNA library) with human fetal brain (Stratagene) as template. Full-length human NEDL1 cDNA was constructed by fusing the PCR-amplified fragment (nucleotide positions 1 (translation start codon) to 68) with KIAA0322 cDNA (donated by T. Nagase of Kazusa DNA Research Institute).

### (Cell culturing and transfection)

The cells were grown in RPMI1640 medium containing 10% heat-inactivated fetal bovine serum (FBS, Life Technologies, Inc.), penicillin (100 IU/ml) and streptomycin (100 µg/ml). COS7 and Neuro2a cells were maintained in Dulbecco's modified Eagle medium: DMEM) containing 10% heat-inactivated fetal bovine serum and penicillin (100 IU/ml)/streptomycin (100 µg/ml). The cells were cultured at 37°C in an atmosphere of moisture-saturated air with 5% carbon dioxide gas. The co-transfection of each expression plasmid was accomplished using LipofectAMINE (Life Technologies, Inc.) according to the manufacturer's instruction manual. For some experiments, the transfected cells were treated for 30 minutes with MG-132 at a final concentration of 40 µM.

### (Yeast Two-Hybrid Screening)

The screening was carried out using a Gal4-based matchmaker two-hybrid system (Clontech) with a cDNA library obtained from human fetal brain (primary screening) and human adult brain (secondary screening). *Saccharomyces cereviciae* CG 1945 cells were transformed with pAS2-1-NEDL1-1 (positions 757-1114, primary screening) or pAS2-1-NEDL1-2 (positions 382-1448, secondary screening). Both of these expression vectors fail to activate only LacZ transcription. The transformants were further transformed with the aforementioned cDNA library. LacZ-positive colonies were selected. Plasmid DNA was extracted from the positive colonies and the nucleic acid sequence was determined.

### (In vitro ubiquitination assay)

An *in vitro* ubiquitination assay was conducted in the following manner. There were incubated 0.5 µg of purified GST fusion protein, 0.25 µg of yeast E1 (Boston Biochem), 1 µl of crude cell lysate from E2s-expressing *E. coli* and 10 µg of bovine Ub (Sigma) in 250 mM Tris-HCl (pH 7.6), 1.2 M NaCl, 50 mM ATP, 10 mM MgCl₂ and 30 mM dithiothreitol. After 2 hours at 30°C, SDS sample buffer was added to suspend the reaction. The sample was analyzed by SDS-PAGE, transferred to a membrane and immunoblotted with anti-ubiquitin monoclonal antibody (Medical Biological Laboratories).

### (Expression construct)

A hemagglutinin-tagged and His6-tagged mammalian ubiquitin expression plasmid was donated by D. Bohmann. The full-length NEDL 1 cDNA was introduced into the mammalian expression plasmid pEF1/His (Invitrogen) or pIRESpuro2 (Clontech). cDNA coding for wild-type or mutant SOD1 was linked to the carboxyl terminal of FLAG or Myc epitope tag sequence and subcloned in pIRESpuro2. FLAG or Myc epitope tag was also linked to the carboxyl terminal of TRAPδ. FLAG or Myc epitope tag was also linked to the amino terminal of Dvl1. The coding sequence was confirmed using an automatic DNA sequencer.

### (Immunoprecipitation and Western blotting analysis)

Anti-NEDL1 antibodies and anti-TRAPδ antibodies were prepared in rabbits against NEDL1 oligopeptide (positions 460-482) and TRAPδ oligopeptide (positions 93-126). In an immunoprecipitation experiment, COS7 cells or Neuro2a cells were co-transfected using the expression plasmids in different combinations. After 48 hours, the cells were lysed in TNE buffer (10 mM Tris-HCl pH 7.8, 150 mM NaCl, 1 % NP-40, 1 mM EDTA, 1 mM PMSF) containing a protease inhibitor cocktail (Sigma). The whole cell lysate was immunoprecipitated using anti-NEDL1 antibody, anti-FLAG antibody (M2, Sigma) or anti-Myc antibody (9B 11, Cell Signaling Technology). The immune complexes were recovered on GTP-binding protein Sepharose beads and boiled in Laemmli sample buffer for elution, electrophoresed on SDS polyamide gel and then transferred to a polyvinylidene difluoride membrane (Immobilon, Millipore) by electroblotting. In a ubiquitination experiment, cytolysis was carried out in RIPA buffer (10 mM Tris-HCl pH 7.4, 150 mM NaCl, 1% NP-40, 0.1% sodium deoxycholate, 0.1% SDS, 1 mM EDTA) and subsequently subjected to high power ultrasonic treatment. The membrane was probed with a first antibody, and then incubated with a second antibody labeled with horseradish peroxidase (Jackson ImmunoResearch Laboratories/Southern Biotechnology Associates, Inc.). The immunoreactive band was detected by ECL enhanced chemiluminescence (Amersham Pharmacia Biotech). In a proteolysis experiment, Neuro2 cells were transfected with a prescribed expression plasmid. At 48 hours after transfection, the cells were treated for a prescribed time with 50 µg/ml of cycloheximide (Sigma). An equivalent of whole cell lysate (50 µg) was then subjected to Western blotting and quantified using Intelligent Quantifier software (Bio Image).

### (Example 1) Binding between NEDL 1 and TRAPδ

COS7 cells were co-transfected with the expression plasmids shown in Fig. 2 (NEDL1 and FLAG-TRAPδ). The whole cell lysate was immunoprecipitated (IP) with anti-FLAG antibody (first panel) or anti-NEDL1 antibody (second panel). The immunoprecipitate was subjected to immunoblotting (IB) using the antibodies shown in the figure. The whole cell lysate was subjected to immunoblot analysis for expression levels of each protein (third and fourth panels). Detection was accomplished using secondary antibody conjugated with horseradish peroxidase. COS7 cells were also transfected with xpress-NEDL1 expressing plasmid for a similar experiment (Fig. 3). The results confirmed binding between NEDL1 and TRAPδ, for exogenous TRAPδ.

### (Example 2) Binding between NEDL1 and mutant SOD1

Whole cell lysate from COS7 cells overexpressing NEDL1 and FLAG-tagged mutant SOD1 or wild-type SOD1 was immunoprecipitated using anti-FLAG antibody (first panel) or anti-NEDL1 antibody (second panel), and then immunoblotted using anti-NEDL1 antibody or anti-FLAG antibody. Expression of NEDL 1 or FLAG-tagged mutant SOD1 was analyzed using anti-NEDL1 antibody (third panel) or anti-FLAG antibody (fourth panel) (Fig. 4). Here it is seen that mutant SOD 1 (C6F, A4V) binds strongly with NEDL1 when onset is followed by a rapid clinical course leading to death of the patient within 1.5 years (Fig. 4, lanes 3,4). On the other hand, there is virtually no binding of mutant SOD1 (L126S, H46R, D90A) with NEDL1 when onset is followed by a slower clinical course (Fig. 4, lanes 11-13). The mutant SOD1 (G93A) which exhibits unique nerve symptoms after onset binds moderately with NEDL 1. Also, it is seen that wild-type SOD 1 and NEDL1 do not bind (no co-precipitation) (lane 2).

### (Example 3) Binding between TRAPδ and mutant SOD 1

COS7 cells were transiently co-transfected with an expression plasmid coding for FLAG-tagged TRAPδ and Myc-tagged mutant SOD1 or Myc-tagged wild-type SOD1. Whole cell lysate was immunoprecipitated using anti-Myc antibody (first panel) or anti-FLAG antibody (second panel), and then immunoblotted using anti-FLAG antibody or anti-Myc antibody. Expression of FLAG-tagged TRAPδ or Myc-tagged mutant SOD1 was analyzed using anti-FLAG antibody (third panel) or anti-Myc antibody (fourth panel). Here it is seen that mutant SOD1 (A4V) binds strongly with TRAPδ when onset is followed by a rapid clinical course leading to death of the patient within 1.5 years (Fig. 7, lane 4). On the other hand, virtually no binding of mutant SOD1 (H46R) with TRAPδ occurs when onset is followed by a slower clinical course (lane 6). Also, it is seen that wild-type SOD1 and TRAPδ do not bind (no co-precipitation) (lane 3).

### (Example 4) NEDL1-dependent ubiquitination

NEDL 1 ubiquitinates mutant SOD 1 in a manner dependent on the type of SOD1. COS7 cells were transiently co-transfected using the expression plasmids shown in Fig. 5. Whole cell lysate from the transfected COS7 cells was immunoprecipitated with anti-Myc antibody, and immunoblotted using anti-ubiquitin antibody (upper panel). The degree of ubiquitination was roughly proportional to the clinical malignancy of FALS (A4V>G93A>H46R). Whole cell lysate was immunoblotted with anti-NEDL1 antibody to confirm expression of the transfected NEDL1 (lower panel). In this photograph, arrows indicate the positions of non-ubiquitinated SOD1, and the molecular weight marker positions are shown at left.

### (Example 5) Half-life of wild-type SOD1 and mutant SOD1 in the presence and in the absence of NEDL1.

Lysates of Neuro2a cells transfected with blank vector or NEDL1 expression plasmid, recovered at different time points after addition of cyclohexidine (final concentration: 50 µg/ml) as shown in Fig. 6, were immunoblotted with anti-FLAG antibody for analysis of SOD 1 protein levels. Mutant SOD1 1 was more rapidly degraded than wild-type SOD1. NEDL1 did not affect degradation of wild-type SOD1. It is seen that degradation of mutant SOD1 protein is accelerated and the half-life of mutant SOD1 protein in the presence of NEDL1 decreases roughly in proportion to the severity of FALS (A4V>G93A>H46R).

### (Example 6) Binding between NEDL1 and Dvl1

Myc-tagged Dvl1 was overexpressed with NEDl1 in COS7 cells. Whole cell lysate was immunoprecipitated with anti-NEDL1 antibody and then immunoblotted using anti-Myc antibody (upper panel of Fig. 8). The expression level of Myc-tagged Dvl1 was analyzed by immunoblotting using anti-Myc antibody (lower panel).

### (Example 7) NEDL1-dependent ubiquitination of Dvl1

NEDL1 ubiquitinates Dvl1 in COS7 cells. COS7 cells were transiently co-transfected using the expression plasmids shown in Fig. 9. Whole cell lysate from the transfected COS7 cells was immunoprecipitated with anti-Myc antibody, and immunoblotted using anti-ubiquitin antibody (upper panel). The whole cell lysate was immunoblotted with anti-xpress-NEDL1 antibody (middle panel) or anti-Myc antibody (lower panel), to confirm expression of the transfected NEDL 1 or Myc-Dvl1.

### (Example 8) Degradation of Dvl1 by NEDL1

Neuro2a cells were transfected using FLAG-tagged Dvl1 expression plasmid, in the presence or in the absence of NEDL1-expressing plasmid. The transfected cells were recovered at different time points after addition of cyclohexidine (final concentration: 50 µg/ml), as shown in Fig. 10. Neuro2a cell lysate was immunoblotted with anti-FLAG antibody for analysis of the Dvl1 protein level. The half -life of FLAG-Dvl1 was notably reduced in the presence of NEDL1.

### (Example 9) Binding between Dvl1 and mutant SOD1

COS7 cells were transiently co-transfected using the expression plasmids shown in Fig. 11. Whole cell lysate was immunoprecipitated with anti-Myc antibody and then immunoblotted using anti-FLAG antibody or anti-Myc antibody. It is seen that binding between Dvl1 and mutant SOD1 is enhanced in the presence of NEDL1 (Fig. 11, lane 4). The binding ability decreased roughly in proportion to the severity of FALS (A4V>G93A>H46R).

### Industrial Applicability

As explained above, the present invention allows determination of clinical malignancy of FALS patients from which mutant SOD1 has been isolated, by evaluating binding ability between NEDL1 and the mutant SOD1 or by evaluating binding ability between NEDL1-associated factors and the mutant SOD1 either in the presence or absence of NEDL1, and it is therefore useful for diagnosis of FALS.

## Claims

1. A method for determining clinical malignancy of FALS, **characterized by** isolating mutant SOD1 from a specimen taken from a FALS patient and evaluating the binding ability between said mutant SOD1 and TRAPδ.

2. A method for determining clinical malignancy of FALS, **characterized by** isolating mutant SOD1 from a specimen taken from a FALS patient and evaluating the binding ability between said mutant SOD 1, and NEDL 1 and Dvl1.

3. A method for determining clinical malignancy of FALS, **characterized by** isolating mutant SOD1 from a specimen taken from a FALS patient and evaluating the binding ability between said mutant SOD 1 and NEDL1.

4. The use of NEDL1 or its substrate for determination of clinical malignancy of FALS.

5. The use of NEDL1 according to claim 4, **characterized by** using isolated mutant SOD1.

6. The use of NEDL1 according to claim 5, **characterized in that** said substrate is TRAPδ or Dvl1.

7. An inhibitor of interaction between mutant SOD1 and NEDL1 and/or its substrate.

8. An inhibitor according to claim 7, **characterized in that** said substrate is TRAPδ or Dvl1.

9. A method of screening for agents that are useful for treatment of FALS, **characterized by** determining whether or not a candidate drug is an inhibitor against interaction between mutant SOD1 and NEDL1 and/or its substrate in neurons.
